Europäisches Patentamt

European Patent Office

Office européen des brevets

(19)

(11) Veröffentlichungsnummer: **0 144 574**
**B1**

(12) **EUROPÄISCHE PATENTSCHRIFT**

(45) Veröffentlichungstag der Patentschrift:
16.08.89

(51) Int. Cl.⁴: **A 61 F 2/32**

(21) Anmeldenummer: 84110987.9

(22) Anmeldetag: 14.09.84

(54) **Hüftendoprothese in Verbundbauweise.**

(30) Priorität: 21.09.83 DE 3334058

(43) Veröffentlichungstag der Anmeldung:
19.06.85 Patentblatt 85/25

(45) Bekanntmachung des Hinweises auf die Patenterteilung:
16.08.89 Patentblatt 89/33

(84) Benannte Vertragsstaaten:
AT BE CH FR GB IT LI LU NL SE

(56) Entgegenhaltungen:
EP-A- 0 086 879
EP-A- 0 086 880
DE-A- 2 319 098
DE-A- 3 123 940
DE-A- 3 212 627
DE-B- 2 537 807
US-A- 3 846 846

(73) Patentinhaber: Witzel, Ulrich, Dr.-Ing., Wittener
Strasse 73d, D-5600 Wuppertal 2 (DE)

(72) Erfinder: Witzel, Ulrich, Dr.-Ing., Wittener Strasse 73d,
D-5600 Wuppertal 2 (DE)

(74) Vertreter: Ostriga, Harald, Dipl.-Ing. et al, Patentanwälte
Dipl.-Ing. Harald Ostriga Dipl.-Ing. Bernd Sonnet
Stresemannstrasse 6-8, D-5600 Wuppertal 2 (DE)

**Beschreibung**

Die Erfindung betrifft eine Hüftendoprothese in Verbundbauweise entsprechend dem Oberbegriff des Hauptanspruchs.

Die im Oberbegriff des Hauptanspruchs 1 beschriebene Hüftendoprothese dieser Gattung ist durch offenkundige Vorbenutzung bekanntgeworden. Bei der bekannten Verbundprothese wird das physiologische und das mechanisch ungünstige Verhältnis der Elastizitätsmoduli von Knochen (Kortikalis und Spongiosa) einerseits und Metallkern andererseits teilweise durch die Zwischenlagerung, d. h. Ummantelung des Metallkerns, gemildert aber nicht aufgehoben. Bei einem ohne Verwendung eines Mantels formschlüssig in den Femur eingebetteten Metallkern hingegen erfolgt die axiale Kraftübertragung einer kragenlosen Prothese ausschließlich am distalen, d. h. unteren Ende des Metallkerns. An dieser Form des tiefen Kraftübertritts von der Prothese in den Femur ändert ggf. auch die Anbringung eines stirnseitig an den Femur angelagerten Prothesenkragens nichts: die Kraft- und Spannungsübertragung bleibt ausschließlich auf den distalen Prothesenbereich beschränkt.

Um einen homogenen Kraftübertritt zwischen Metallkern und Mantel erreichen zu können, müßte der Metallquerschnitt, z. B. bei der Verwendung von Stahl, im Verhältnis zum Kunststoff auf etwa $1/_{67}$ reduziert werden. Eine solche Reduzierung würde aber praktisch an der dynamischen Biegemomentenbelastbarkeit und den sich daraus zwangsweise ergebenden inneren Implantatbrüchen scheitern. Die als Gegenreaktion hierauf inzwischen ständig vergrößerten Metallkernquerschnitte, die eine sichere Momentenaufnahme gewährleisten sollen, führten schließlich das Prinzip von sogenannten isoelastischen Prothesen ad absurdum.

Durch die US-A-3 846 846 ist eine Hüftendoprothese bekannt, bei welcher ein etwa gewindestangenartiger Metallkern, der einen distalen rundlichen Verdrängungskörper aufweist, etliche axial hintereinandergeschaltete, einander in Axialrichtung überlappende konische Spreizhülsen durchsetzt. Bei Axialverspannung des Metallkerns bewirkt dessen endseitiger distaler Verdrängungskörper einen sich über die Axiallänge der Prothese erstreckenden Spreizdübeleffekt, der keinesfalls einer physiologischen proximalen Einleitung der axialen Hüftkraftkomponente förderlich ist.

Von einer Endoprothese, beispielsweise einer Kniegelenkprothese, gemäß der EP-A-86 880 (s. a. EP-A-86 879) ist es bekannt, einen Metallkern invarianten Querschnitts innerhalb eines im Markhohlraum eines Röhrenknochens festgelegten Führungsstückes mit axialer Gleitpassung zu lagern.

Im Bewußtsein der Verbesserungsbedürftigkeit der eingangs beschriebenen und durch offenkundige Vorbenutzung bekanntgewordenen Hüftendoprothese dieser Gattung, liegt der Erfindung die Aufgabe zugrunde, im Sinne einer physiologischen Krafteinleitung die Axialkomponente der Hüftgelenkkraft coxal, d. h., gelenknah, und die Momentenkompensation distal, d. h. gelenkfern, vorzunehmen. Hierdurch soll eine ständige Belastung, insbesondere Axialbelastung, der proximalen Drucktrabekel erfolgen und eine Knochenatrophie vermieden werden.

Zusätzlich muß eine radiale und axiale Relativbewegung zwischen der Knochensubstanz und der Prothesenoberfläche oder, bei noch gebräuchlicher Knochenzementeinbettung, zwischen Knochen und Zement oder zwischen Prothese und Zement unter allen Umständen vermieden werden, da sonst Prothesenauslockerungen zu erwarten sind.

Die vorbezeichnete Aufgabe wurde erfindungsgemäß entsprechend dem Kennzeichenteil des Hauptanspruchs gelöst.

Die erfindungsgemäße spezielle Gestaltung der Hüftendoprothese in Verbundbauweise erstmals in Form einer Gleitlagerprothese führt zu einem klinisch geforderten Dauerimplantat. Mit der erfindungsgemäßen Gleitlagerprothese ist eine weitestgehend angenäherte physiologische Krafteinleitung von der Hüftpfanne in den Femur unter Vermeidung der bisherigen Knochenatrophie am calcar femorale erzielt worden. Hierdurch werden die bei herkömmlichen Prothesenimplantationen üblichen Auslockerungsraten vermieden, die physisch belastende und kostspielige Reoperationen zur Folge haben. Die erfindungsgemäße Hüftendoprothese führt daher insbesondere bei gelenkerkrankten jüngeren Menschen zu einer Indikationserweiterung.

Und zwar erfolgt bei der erfindungsgemäßen Gleitlagerprothese die axiale Krafteinleitung in den Femur vollständig coxal über ein im proximalen Bereich angeordnetes Festlager nach biologischem Vorbild. Zugleich ist der distale Bereich des Prothesenstiels spielfrei in einem radialen Stützlosläger nach Art einer – relativ auf den Knochen bezogen – mittelbaren Gleiteinbettung durchgeführt. Hiermit wird zugleich der Vorteil einer möglichst reinen Querkraftübertragung zur notwendigen Momentenkompensation erzielt.

Weitere Erfindungsmerkmale ergeben sich aus den Unteransprüchen sowie aus den Zeichnungen in Verbindung mit der Zeichnungsbeschreibung.

In den Zeichnungen ist die Erfindung anhand bevorzugter Ausführungsbeispiele näher dargestellt, er zeigen

Fig. 1 einen schematischen Längsschnitt durch eine in den Femur (Oberschenkelknochen) implantierte Hüftendoprothese,

Fig. 2 ein erstes konkretes Ausführungsbeispiel in Anlehnung an die Darstellungsweise gemäß Fig. 1,

Fig. 3 die auf ein Kraftaufnahmeschema reduzierte Darstellung gemäß Fig. 1,

Fig. 4 ein zweites Ausführungsbeispiel in der Darstellungsweise gemäß Fig. 2,

Fig. 5 eine Teilansicht des distalen Stielbereichs gemäß den Ausführungsformen in Fig. 2 oder 4,

Fig. 6 lediglich den oberen Bereich gemäß Fig. 4 in einer abgewandelten Form,

Fig. 7–11 unterschiedliche schematische Darstellungen weiterer unterschiedlicher Ausführungsformen.,

Fig. 12 vier Alternativschnitte u–x etwa entsprechend der Schnittlinie XII–XII in Fig. 7 und

Fig. 13 eine Einzelheit, wie in Fig. 8 mit der Bezugsziffer XIII kenntlich gemacht.

In den Zeichnungen ist eine Hüftendoprothese in Verbundbauweise allgemein mit der Bezugsziffer 10 bezeichnet.

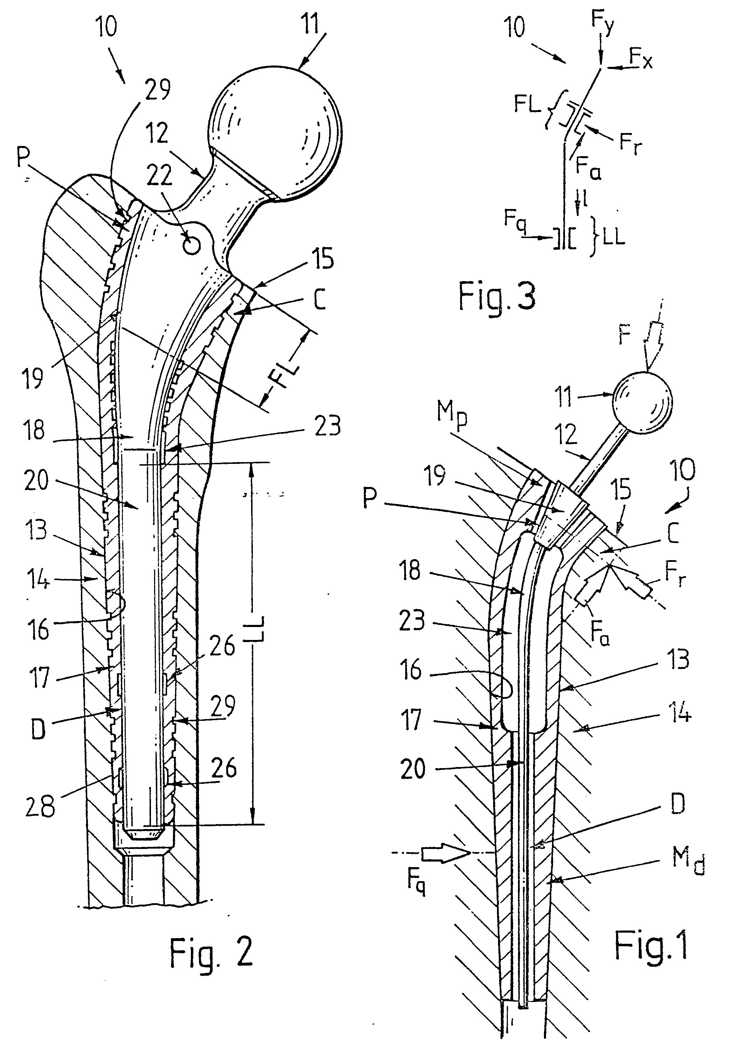

Der grundsätzliche Aufbau sowie die Funktion einer Hüftendoprothese ist anhand der schematischen Darstellung gemäß Fig. 1 näher erläutert. Hiernach weist die Hüftendoprothese 10 eine Gelenkkugel 11, einen sich daran anschließenden Prothesen-Hals 12 und einen Stiel 13 auf, welcher im Oberschenkel, dem sogenannten Femur 14, aufgenommen ist.

Die nach oben weisende Stirnfläche des Femurs 14, an welche sich zuvor der natürliche Schenkelhals angeschlossen hatte, ist mit 15 bezeichnet.

Der Stiel 13 der Hüftendoprothese 10 ist satt in einer Höhlung 16 des Femurs 14 aufgenommen.

Der Stiel 13 weist einen äußeren Mantel 17 auf, welcher aus Kunststoff besteht und einen insgesamt mit 18 bezeichneten Metallkern, z. B. aus Stahl, auf mindestens einem Teil seiner axialen Länge, beim Beispiel gemäß Fig. 1 auf ganzer Länge, umgibt.

In seinem oberen – dem proximalen – Bereich P bildet der Metallkern 18 einen sich von der Gelenkkugel 11 weg verjüngenden konusartigen Teilbereich 19, während der untere Bereich, der distale Bereich D, im weitesten Sinne ein Zylinderbauteil 20 darstellt, welches beim Ausführungsschema gemäß Fig. 1 auf ganzer Länge einen gleichbleibenden Zylinderquerschnitt besitzen oder auch in gestufte Zylinderbereiche unterteilt sein kann.

Der gesamte Mantel 17 ist mit seinem peripheren Bereich fest mit der Höhlung 16 des Femurs 14 verbunden, insbesondere dort in zweckentsprechender Weise adhäsiv befestigt, wie z. B. einzementiert. Hinzu kommen noch Formschlußverbindungsmittel, wie sie noch im einzelnen insbesondere anhand der Fig. 2 erklärt werden.

Der obere bzw. proximale Bereich des Mantels 17 ist allgemein mit der Bezeichnung $M_p$ und der untere, der distale Bereich des Mantels 17, mit der Bezeichnung $M_d$ versehen.

Der proximale Bereich P des Metallkerns 18 ist, zweckmäßig durch Formschluß, allseitig fest vom proximalen Mantelbereich $M_p$ umschlossen. Der proximale Bereich P des Metallkerns 18 bildet demnach unter Zwischenschaltung des proximalen Mantelbereichs $M_p$ ein Festlager FL, welches auch aus Fig. 3 ersichtlich ist.

Während die proximalen Bereiche P und $M_p$ Bestandteil des vorbezeichneten Festlagers FL sind, stellen der distale Bereich D des Metallkerns 18 gemeinsam mit dem distalen Mantelbereich $M_d$ ein Loslager LL in Form eines in axialer Richtung 1 beweglichen Gleitlagers dar. Das Los- bzw.

Gleitlager LL und die Achsrichtung 1 sind ebenfalls aus Fig. 3 zu ersehen.

Der Kräfteverlauf (s. Fig. 1 und 3) ist folgender: die über das Hüftgelenk in die Gelenkkugel 11 eingeleitete Gesamtkraft, die Hüftkraft, ist als resultierende Kraft in Fig. 1 mit F bezeichnet. Die Hüftkraft F resultiert aus einer Vertikalkomponente $F_y$ und aus einer Horizontalkomponente $F_x$.

Die Vertikalkomponente $F_y$ wird vollständig im Festlager FL aufgenommen und es entstehen die Lagerkraftkomponenten $F_a$ und $F_r$. Hierbei bezeichnet der Index «a» die axiale Kraftkomponente, während der Index «r» für die radiale Kraftkomponente steht.

Das aus der Hüftkraft F resultierende Biegemoment wird durch das Loslager LL, also zwischen dem zylindrischen Teil 20 im distalen Bereich D des Metallkerns 18 und dem distalen Mantelbereich $M_d$ kompensiert. Die hierbei auftretende Lagerkraft ist als Querkraft mit $F_q$ bezeichnet.

Mit der Anordnung, wie in den Fig. 1 und 3 grundsätzlich dargestellt, wird folgender vorteilhafter Effekt erzielt: dadurch, daß die Kraftkomponenten $F_a$ und $F_r$ vollständig im Bereich des Festlagers FL abgebaut und damit im wesentlichen auf den stirnseitigen Axialbereich bei 15 auf den Femur 14 übertragen werden, ist der Femur 14 in diesem Bereich C, dem sogenannten calcar femorale, vornehmlich durch die Axialkraftkomponente $F_a$ ständig aktiviert. Diese durch andauernde Schwellbelastung bedingte ständige Aktivierung vermeidet mit weitestgehender Sicherheit eine sonst bei derartigen Hüftimplantaten stets zu besorgende Atrophie (biologischer Abbau der Knochensubstanz).

Während, wie beschrieben, die Axialkraftkomponente $F_a$ und die Radialkraftkomponente $F_r$ völlig im proximalen Bereich des Femurs 14, d. h. im calcar femorale, abgebaut werden, braucht der distale Bereich des Femurs, d. h. in Höhe der Bereiche $M_d$ und D, lediglich Gleitlagerkräfte in Form einer Querkraftkomponente $F_q$ aufzunehmen. Die in diesem Bereich eintretende Axialbewegung des zylindrischen Bereichs 20 des Metallkerns 18 wird durch die Gleitlageranordnung toleriert, so daß die Einleitung einer jeglichen schädlichen Axialkraft (in Richtung 1) im distalen Bereich D des Metallkerns 18 entfällt. Zu erklären bleibt noch, daß die axiale Relativbewegung des Zylinderbauteils 20 (distaler Bereich D) bezüglich des Mantelbereichs $M_d$ und des damit fest verbundenen Knochengerüstes bedingt ist durch die unterschiedlichen Elastizitätsmoduli des Mantelkunststoffes 17 sowie des Knochengerüstes 14 einerseits und der Metalleinlage 18 andererseits. Das Phänomen einer schädlichen Axialbewegung im Tiefenbereich der Einbettung tritt grundsätzlich bei allen bisher üblichen Metallimplantaten dieser Gattung auf. Bei den dargestellten erfindungsgemäßen Ausführungsformen hingegen ist diese Axialbewegung der Metalleinlage 18 neutralisiert und deshalb unkritisch und unschädlich.

Hingegen vollzieht sich die Krafteinleitung bei der eingangs beschriebenen Hüftendoprothese dieser Gattung so: der Abbau der Axialkraft geschieht im Tiefenbereich des Femurs, während der proximale Bereich des Femurs, also das calcar femorale, keinerlei Axialkräfte aufzunehmen hat. Das Fehlen der Axialkräfte im Bereich des calcar femorale bedingt aber mit Sicherheit auf kürzere oder fernere Sicht eine Atrophie, die bei den erfindungsgemäßen Ausführungsformen gerade vermieden ist.

Bei der eingangs beschriebenen Hüftendoprothese dieser Gattung kann dieser Mangel auch nicht durch externe Anordnung eines Kragens vor der proximalen Stirnfläche des Femurs behoben werden. Mit einer solchen Anordnung ist beabsichtigt, die Stirnseite des Femurs in dessen proximalem Bereich im Sinne einer Druckaktivierung zu beaufschlagen. Eine solche axiale Druckbeaufschlagung findet aber nach neueren Untersuchungen in Wirklichkeit nicht statt. Vielmehr werden nach wie vor die Axialkräfte mit den vorbezeichneten nachteiligen Folgen vollständig im Tiefenbereich des Femurs abgebaut, während dessen proximaler Bereich unaktiviert verbleibt und deshalb nach und nach atrophiert. Die nachteilige Folge ist eine Auslockerung. Die ausgelockerte Verbindungsstelle ist aber weniger in der Lage, Kräfte zu übertragen, und hat eine Verkürzung der wirksamen Einspann- bzw. Einbettungslänge zum Ergebnis, woraus wiederum eine Erhöhung der Reaktionskräfte resultiert. In schädlicher Weise tritt hinzu der konzentrierte Kraftübertritt im distalen Bereich der Prothese, verbunden mit der durch unterschiedliche Elastizitätsmoduli bedingten axialen Relativbewegung des distalen Metallkerns. Dieser Fehler zeigt sich im übrigen unabhängig davon, ob eine Ganzmetallprothese oder eine Verbundprothese, letztere mit distaler fester Ummantelung, zur Verwendung gelangt. In jedem Falle sind diese bekannten Prothesen im gesamten Längenbereich der Prothese, also auch in deren distalem Bereich, formschlüssig allseitig fest mit dem Femur verbunden.

Allgemein gilt, daß die in Fig. 1 benutzten und in den übrigen Fig. wiederkehrenden gleichen Bezugsziffern analoge Bereiche bzw. Elemente gleicher oder vergleichbarer Funktion betreffen.

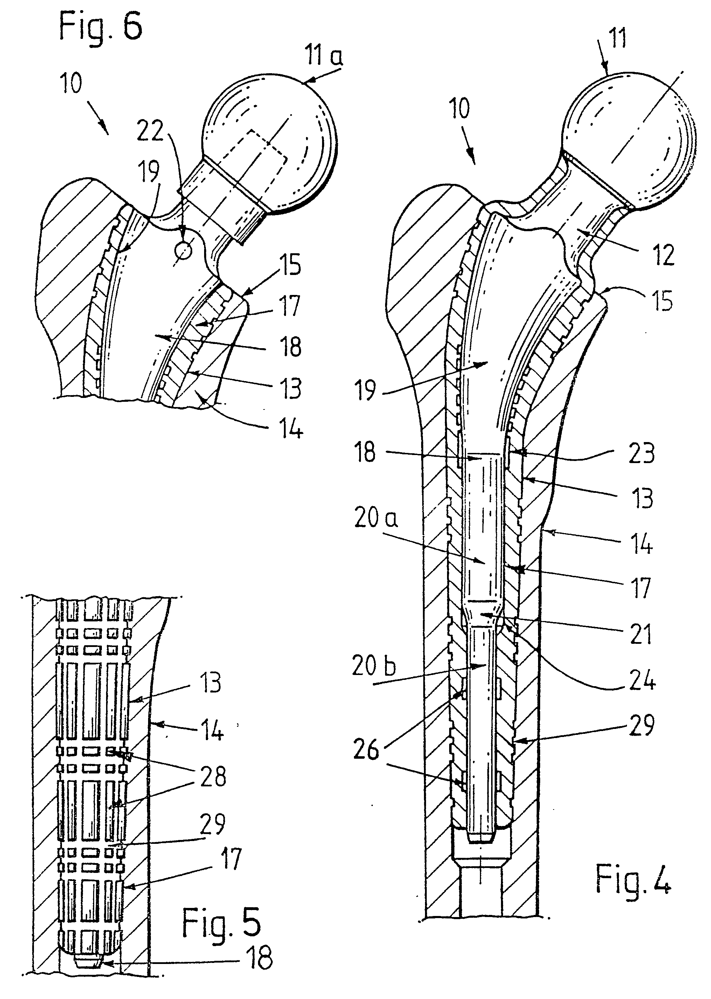

Die in den Fig. 2 und 4 dargestellten Ausführungsformen sind im wesentlichen gleich. Die Unterschiede bestehen darin, daß gemäß Fig. 2 der distale Bereich D des Metallkerns 18 ein Zylinderbauteil 20 mit durchgehend gleichbleibendem Querschnitt bildet, während das Zylinderbauteil 20 gemäß Fig. 4 zwei abgestufte Zylinderbauteile 20a, 20b mit einem im wesentlichen konischen Übergangsbereich 21 aufweist. Diese Abstufung erfolgt entsprechend dem Verlauf des eingeleiteten Biegemomentes. Ein weiterer Unterschied zwischen den Ausführungsformen gemäß den Fig. 2 und 4 besteht darin, daß gemäß Fig. 4 der Kunststoffmantel 17 proximal so verlängert ist, daß er zugleich den Prothesenhals 12 umkleidet. Diese Umkleidung des Prothesenhalses 12 fehlt gemäß Fig. 2. Die Ausführungsformen gemäß Fig. 2 (vgl. ebenfalls Fig. 6) erleichtert daher eine Nachoperation für den Fall, daß der Metallkern 18 im Stiel- oder im Gelenkbereich aus irgendeinem Grunde Schaden nehmen sollte. Bei einer aus diesem Grunde erforderlichen Nachoperation würde es demnach genügen, lediglich die Metalleinlage 18 zu entfernen, während der Kunststoffmantel 17 in der Höhlung 16 des Femurs 14 verbleiben könnte. Zur Erleichterung der Herausnahme des Metallkerns weist dieser im Bereich seines Konusbereichs 19 eine Handhabungsöffnung 22 auf (s. ebenfalls Fig. 6).

Der Konus 19 des Metallkerns 18 ist hierbei entsprechend den Fig. 2 und 6 – ebenso wie die Innenmantelfläche des Mantels 17 – im proximalen Bereich so ausgebildet, daß eine Herausnahme des gesamten Metallkerns 18 nur gegen den Widerstand einer Schnappverrastung aus dem fest implantierten Mantel 17 möglich ist.

Ergänzend bleibt noch hervorzuheben, daß zwischen dem proximalen Bereich $M_p$ und dem distalen Bereich $M_d$ des Mantels 17 ein Freiraum 23, beispielsweise in Form einer Ringnut, vorhanden ist. Der Freiraum 23 bietet die Gewähr, daß keinesfalls vom Formschlußbereich des Festlagers FL axiale Kraftkomponenten in den unteren Bereich $M_d$ des Mantels 17 übergeleitet werden. Der Freiraum 23 stellt daher eine Zäsur zwischen den beiden Lagerstellen FL und LL dar.

Ein Freiraum 24 ähnlicher Funktion umgibt gemäß Fig. 4 den konischen Übergangsbereich 21 zwischen den beiden zylindrischen Teilbereichen 20a und 20b. Durch diesen Freiraum 24 wird ebenfalls eine schädliche axiale Kraftübertragung im Bereich der Abstufung 20a und 20b auf den umgebenden distalen Bereich $M_d$ des Mantels 17 ausgeschlossen.

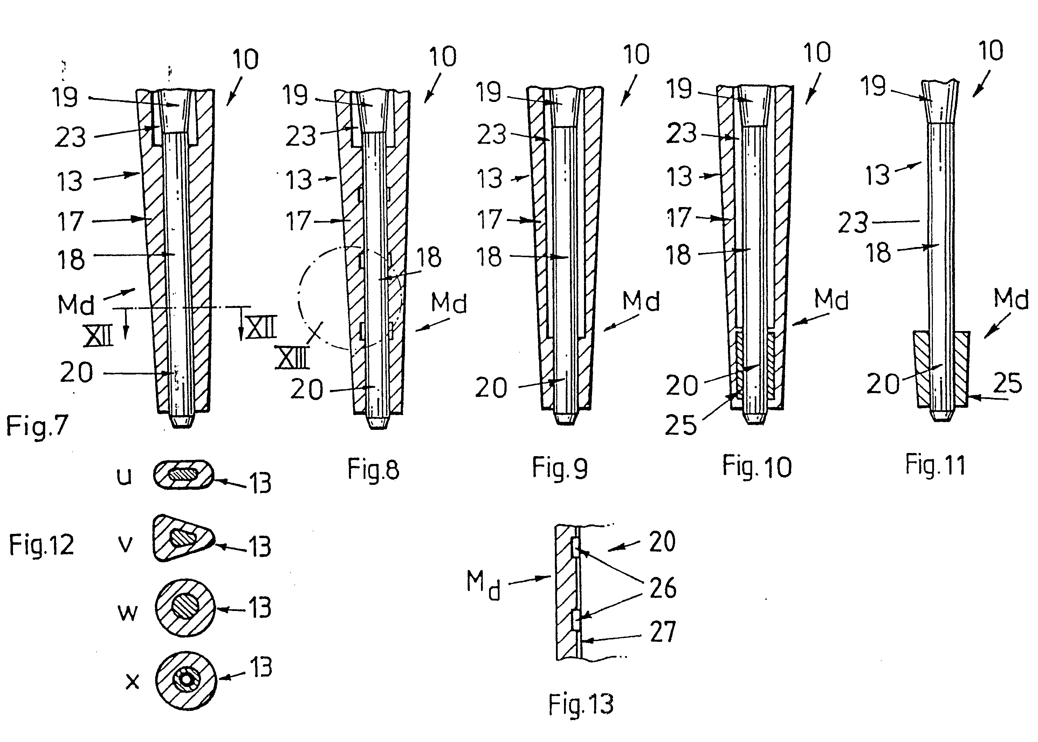

Wie aus den Fig. 7–11 zu ersehen, können die Freiräume 23 zur Definition der axialen Länge des Gleit- bzw. Loslagers LL eine unterschiedliche axiale Ausdehnung besitzen. Dieses kann so weit gehen, daß der Freiraum 23 eine Unterbrechung des Mantels 17 bildet, so daß dieser aus zwei voneinander axial distanzierten Teilmantelbereichen besteht. In diesem Zusammenhang wird verwiesen auf Fig. 11, wonach der distale Bereich $M_d$ des Mantels 17 von einem axialen Teilmantelbereich 25 gebildet ist. Der proximale Mantelbereich $M_p$, welcher ebenfalls ein diskretes Bauteil bildet, ist aus Gründen einer Zeichnungsvereinfachung in Fig. 11 nicht gezeigt.

Zusätzliche im Bereich LL vorgesehene Ringnuten 26 an den Innenmantelfläche des distalen Mantelbereichs $M_d$ können – ebenso wie die Freiräume 23, 24 – mit einem Gleitmittel zur Herabsetzung des Reibungsbeiwertes gefüllt sein.

Der distale Mantelbereich $M_d$ kann mantelaußenseitig etwa so beschaffen sein, wie in Fig. 5 (s. a. Fig. 2 und 4) dargestellt. Er kann demgemäß eine durch Rippen bzw. Nocken 28 und durch Nuten bzw. Aussparungen 29 geprägte Struktur erhalten, welche dem formschlüssigen Verbund mit dem Femur 14 dienen soll. Eine derartige Struktur der Außenfläche des Mantels 17 führt – wie in ähnlicher Weise bekannt – zu einem postoperativen biologischen Verbund zwischen der Höhlung 16 des Femurs 14 und der Außenfläche des Mantels 17.

Lediglich der Vollständigkeit halber wurde gemäß Fig. 6 noch eine an sich bekannte Variante dargestellt, gemäß welcher die Gelenkkugel 11 ein metallisches oder nichtmetallisches (z. B. Keramik) lösbar zu befestigendes Bauteil 11a bildet.

Aus Fig. 10 ist im übrigen ersichtlich, daß der distale Mantelbereich $M_d$ wiederum von einem separaten Bauteil 25 gebildet ist, welches indes – im Unterschied zur Darstellung gemäß Fig. 11 vom Kunststoff des Mantels 17 umgeben und axial gesichert ist. Beide Bauteile 25 gemäß Fig. 10 und Fig. 11 können auch aus Keramik bestehen.

Zur Herabsetzung der Gleitreibung können die Innenwand des Kunststoffmantels 17 und/oder die Außenfläche des zylindrischen Bereichs 20 des Metallkerns 18 mit einem die Gleitreibung herabsetzenden Werkstoff, z. B. mit TFE oder mit PTFE, beschichtet sein (Schicht 27 gemäß Fig. 13).

Anhand von Fig. 12 soll noch verdeutlicht werden, daß entsprechend den einzelnen Querschnittsdarstellungen u bis x die distalen Bereiche D und $M_d$ unterschiedlich gestaltet sein können, aber immerhin ein Zylinderbauteil 20 im weitesten Sinne bilden.

## Patentansprüche

1. Hüftendoprothese (10) in Verbundbauweise mit einer Gelenkkugel (11, 11a) und einem an dieser über einen Hals (12) befestigten Stiel (13), welcher in ein zu seinem proximalen Ende hin konisch erweiternd ausgenommenes Femur (14) einsetzbar und auf mindestens einem Teilbereich seiner Einbettungslänge allseitig fest mit dem Femur (14) über einen aus Kunststoff od. dgl. bestehenden äußeren Mantel (17) verbindbar ist, der von einem sich über die gesamte Einbettungslänge erstreckenden Metallkern (18) durchsetzt und mit diesem auf mindestens einem axialen Teilbereich (FL) allseitig fest verbunden ist, dadurch gekennzeichnet, daß der proximale Bereich (P) des Metallkerns (18) zur Gelenkkugel (11, 11a) hin konisch erweitert ist und daß der distale Bereich (D) des Metallkerns (18) zylindrisch ausgebildet ist, daß nur der proximale Bereich (P) des Metallkerns (18) allseitig fest mit einem proximalen Mantelbereich ($M_p$), ein Festlager (FL) bildend, verbunden ist, während der distale Bereich (D) des Metallkerns (18), ein Loslager (LL) bildend, mit axialer Gleitpassung in einem distalen Mantelbereich ($M_d$) gelagert ist, und daß der Metallkern (18) im Übergang vom Festlager (FL) zu seinem dem Gleit- bzw. Loslager (LL) zugehörigen Bereich (D) mit allseitigem Umfangsspiel, z. B. in Gestalt einer Ringnut, von mindestens einem mantelseitigen Freiraum (23) umgeben ist.

2. Hüftendoprothese nach Anspruch 1, dadurch gekennzeichnet, daß der Metallkern (18) im Bereich seiner Gleitlagerung (bei D bzw. LL) mehrere koaxial ineinandergefügte, gegeneinander gleitbare Metallkörper bildet.

3. Hüftendoprothese nach Anspruch 1 oder nach Anspruch 2, dadurch gekennzeichnet, daß der Freiraum (23) über einen erheblichen axialen Längenbereich des Metallkerns (18) zur Bildung eines relativ kurzen Gleitlagers (LL) ausgedehnt ist.

4. Hüftendoprothese nach einem der Ansprüche 1 bis 3, dadurch gekennzeichnet, daß der Freiraum (23) von einer Lücke zwischen dem proximalen ($M_p$) und dem distalen ($M_d$) Mantelbereich gebildet ist.

5. Hüftendoprothese nach einem der Ansprüche 1 bis 4, dadurch gekennzeichnet, daß der distale Mantelbereich ($M_d$) von einer ggf. von Kunststoff umgebenen Lagerbüchse (25), z. B. aus keramischem Werkstoff, gebildet ist.

6. Hüftendoprothese nach einem der Ansprüche 1 bis 5, dadurch gekennzeichnet, daß im distalen Innenmantelbereich (bei $M_d$) im Axialabstand voneinander Ringnuten (26) vorgesehen sind.

7. Hüftendoprothese nach einem der Ansprüche 1 bis 5, dadurch gekennzeichnet, daß der Freiraum und/oder die Ringnuten mit einem Gleitmittel gefüllt sind.

8. Hüftendoprothese nach einem der Ansprüche 1 bis 7, dadurch gekennzeichnet, daß mindestens eine Gleitlagerfläche (27) aus einem die Reibung herabsetzenden Werkstoff, z. B. TFE oder PTFE, besteht.

9. Hüftendoprothese nach einem der Ansprüche 1 bis 8, dadurch gekennzeichnet, daß in Anpassung an den Biegemomentenverlauf der Metallkern (18) zu seinem distalen Ende hin einen stufenweise verminderten Querschnitt aufweist.

10. Hüftendoprothese nach einem der Ansprüche 1 bis 9, dadurch gekennzeichnet, daß der Metallkern (18) gegen den Widerstand einer Schnappverrastung aus dem fest implantierbaren Mantel (17) herausziehbar ist.

## Claims

1. A hip endoprosthesis (10) of composite construction with a joint ball (11, 11a) and fastened thereto via a neck (12) a stem (13) which may be inserted into a femur (14) recessed at its proximal end in a widening taper, and may over at least a partial zone of its embedded length be connected firmly to the femur (14) via an outer jacket (17) consisting of plastics or the like, through which passes a metal core (18) extending over the whole embedded length and which is connected firmly all round to the latter over at least a partial axial zone (FL), characterized in that the proximal zone (P) of the metal core (18) is widened in a taper towards the joint ball (11, 11a) and that the distal zone (D) of the metal core (18) is made cylindrical, that only the proximal zone (P) of the metal core (18) is connected firmly all round to a proximal zone ($M_p$) of the jacket to form a solid bearing (FL) whilst the distal zone (D) of the metal core (18), forming a movable bearing (LL), is supported with an axial sliding fit in a distal zone ($M_d$) of the jacket, and that the metal core (18) at the transition from the solid bearing (FL) to its zone (D) belonging to the sliding or movable bearing (LL) is surrounded by at least one free space (23) next the jacket with circumferential play all round, e. g., in the shape of an annular groove.

2. A hip endoprosthesis as in Claim 1, characterized in that the metal core (18) in its slide-bearing zone (at D resp. LL) forms a number of metal bodies joined coaxially to be able to slide against one another.

3. A hip endoprosthesis as in Claim 1 or as in Claim 2, characterized in that the free space (23) is spread out over a considerable axial zone of the length of the metal core (18) for the formation of a relatively short sliding bearing (LL).

4. A hip endoprosthesis as in one of the Claims 1 to 3, characterized in that the free space (23) is formed by a gap between the proximal ($M_p$) and distal ($M_d$) zones of the jacket.

5. A hip endoprosthesis as in one of the Claims 1 to 4, characterized in that the distal zone ($M_d$) of the jacket is formed by a bearing bush (25) of, e. g., ceramic material surrounded if necessary by plastics.

6. A hip endoprosthesis as in one of the Claims 1 to 5, characterized in that in the distal zone (at $M_d$) inside the jacket annular grooves (26) are provided at axial intervals.

7. A hip endoprosthesis as in one of the Claims 1 to 5, characterized in that the free space and/or the annular grooves are filled with a lubricant.

8. A hip endoprosthesis as in one of the Claims 1 to 7, characterized in that at least one sliding bearing area (27) consists of a material which reduces the friction, e. g., TFE or PTFE.

9. A hip endoprosthesis as in one of the Claims 2 to 8, characterized in that in adaptation to the trend of the bending moment the metal core (18) exhibits towards its distal end a cross-section reduced in stages.

10. A hip endoprosthesis as in one of the Claims 1 to 9, characterized in that the metal core may be extracted against the resistance of a snap location from the solidly implantable jacket (17).

## Revendications

1. Endoprothèse (10) coxale ou coxofémorale de construction composite, avec une tête d'articulation (11, 11a) et une tige (13) qui, fixée à cette dernière par l'intermédiaire d'un collet (12), peut être engagée dans un fémur (14) évidé d'une manière coniquement évasée en direction de son extrémité proximale et peut être solidairement liée de tous les côtés au fémur (14) sur au moins une partie de la zone de sa longueur d'insertion par l'intermédiaire d'une enveloppe (17) extérieure qui se compose de matière plastique ou similaire, est traversée de part en part par un noyau métallique (18) s'étendant sur la totalité de la longueur d'insertion et est solidairement liée de tous les côtés à ce dernier sur une zone partielle axiale (FL), caractérisée par le fait que la zone proximale (P) du noyau métallique (18) est coniquement évasée en direction de la tête d'articulation (11, 11a) et que la zone distale (D) du noyau métallique (18) est réalisée dans une forme cylindrique, que seule la zone proximale (P) du noyau métallique (18) est solidairement liée de tous les côtés avec une zone proximale ($M_p$) de l'enveloppe pour former une assise fixe (FL), tandis que la zone distale (D) du noyau métallique (18) formant une assise lâche (LL) et disposée, moyennant un ajustage autorisant un glissement axial, dans une zone distale ($M_d$) de l'enveloppe, et que le noyau métallique (18) est entouré dans la transition allant de l'assise fixe (FL) à sa zone (D) faisant partie de l'assise lâche (LL) ou coulissante avec un jeu sur tous les côtés de sa périphérie, par exemple sous la forme d'une rainure annulaire, par au moins un espace libre (23) ménagé côté enveloppe.

2. Endoprothèse coxale selon la revendication 1, caractérisée par le fait que le noyau métallique (18) forme, dans la zone de son assise coulissante (au niveau de D ou LL) plusieurs corps métalliques qui sont jointivement assemblés les uns dans les autres dans un sens coaxial et peuvent glisser les uns par rapport aux autres.

3. Endoprothèse coxale selon la revendication 1 ou 2, caractérisée par le fait que l'espace libre (23) est étendu sur une zone longitudinale axiale importante du noyau métallique (18) pour former un palier à glissement (LL) relativement court.

4. Endoprothèse coxale selon l'une des revendications 1 à 3, caractérisée par le fait que l'espace libre (23) est formé par une discontinuité entre la zone proximale ($M_p$) et la zone distale ($M_d$) de l'enveloppe.

5. Endoprothèse coxale selon l'une des revendications 1 à 4, caractérisée par le fait que la zone distale ($M_d$) de l'enveloppe est formée par une douille d'assise (25), par exemple en un matériau céramique, ceinturée le cas échéant par de la matière plastique.

6. Endoprothèse coxale selon l'une des revendications 1 à 5, caractérisée par le fait que dans la zone distale de l'enveloppe interne (en $M_d$) sont prévues des rainures annulaires (26) axialement espacées l'une de l'autre.

7. Endoprothèse coxale selon l'une des revendications 1 à 5, caractérisée par le fait que l'espace libre et/ou les rainures annulaires sont remplies d'un agent lubrifiant.

8. Endoprothèse coxale selon l'une des revendications 1 à 7, caractérisée par le fait qu'au moins l'une (27) des surfaces du palier à glissement est constituée par une matière d'œuvre abaissant la friction, par exemple par du TFE ou du PTFE.

9. Endoprothèse coxale selon l'une des revendications 1 è 8, caractérisée par le fait que, s'adaptant à l'allure des moments de flexion, le noyau métallique (18) présente, par rapport à son extrémité distale, une section transversale diminuant par paliers.

10. Endoprothèse coxale selon l'une des revendications 1 à 9, caractérisée par le fait que le noyau métallique (18) peut être extrait, à l'encontre de la résistance d'un crantage par déclic, de l'enveloppe (17) fixement implantée.

Fig. 2

Fig. 3

Fig.1

Fig. 6

Fig. 5

Fig. 4

Fig.7

Fig.8

Fig.9

Fig.10

Fig.11

Fig.12

u ← 13

v ← 13

w ← 13

x ← 13

Fig.13

EP 0 144 574 B1